# EUROPEAN PATENT APPLICATION

(11) **EP 1 623 713 A1**
(43) Date of publication of application: **08.02.2006**
(21) Application number: 04291756.7
(22) Date of filing: 09.07.2004
(51) Int. Cl.: A61K 31/58, A61K 31/56

(54) **Cominations of pure anti-estrogen with aromatase inhibitors**

(71) Applicant: Proskelia SAS, 92230 Romainville (FR)
(72) Inventor: Clement-Lacroix, Philippe, 93310 Le Pre Saint Gervais (FR); Nique, Francois, 94170 Le Perreux sur Marne (FR)
(74) Representative: Hirsch & Associés

(57) **Abstract**

Combinations of pure anti-estrogens, with aromatase inhibitors.

The pure anti-estrogen can be a 19-nor steroid having a thiocarbonated chain in position 11β.

The activity of the aromatase inhibitor is potentiated by the pure anti-estrogen.

## Description

The invention relates to combinations of pure anti-estrogen with aromatase inhibitors. More specifically it relates to combinations of a pure anti-estrogen 19-nor steroid having a thiocarbonated chain in position 11β with aromatase inhibitors. Such combinations have a surprisingly potent effect on breast cancer.

Some approaches of combinations of aromatase inhibitors with anti-estrogen such as tamoxifen have been tried, but have been revealed unsuccessful in that they did not increase the efficacy compared to the aromatase inhibitor alone : Q. Lu, Y. Liu et al, Breast Cancer Research and Treatment, 57, 183-192 (1999). Also it is reported in WO 02/30429 that attempts to combine an anti-estrogen with an inhibitor of sex steroid hormone synthesis such as an aromatase inhibitor have failed. Furthermore it is explained that drug-drug interactions can adversely impact safety and/or effectiveness.

European patent EP 623140 describes 19-nor steroids having a thiocarbonated chain in position 11β and derivatives of the formula : 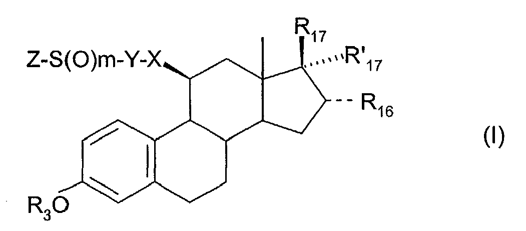 in which
R₁₇ and R'₁₇ are such that:
either R₁₇ and R'₁₇ together form a ketone function, an oxime function, a hydrazono function or a methylene radical,
or R₁₇ is a hydroxyl radical, a hydroxymethyl radical or an acyloxy radical having at most 12 carbon atoms and R'₁₇ represents a hydrogen atom, an alkyl, alkenyl or alkynyl radical having at most 8 carbon atoms, each of these substituents being optionally substituted,

R₃ represents a hydrogen atom, a linear or branched alkyl radical or a cyclic alkyl radical having at most 8 carbon atoms or an acyl radical having at most 12 carbon atoms,
R₁₆ represents a hydrogen atom, a halogen atom or an alkyl radical having at most 8 carbon atoms,
m has the value 0, 1 or 2,
X, Y and Z are such that:
X represents a methylene radical, an arylene or arylenoxy group, having at most 10 carbon atoms linked to the steroid by a carbon atom,
Y represents a saturated or unsaturated, linear or branched aliphatic chain, containing 1 to 18 carbon atoms, optionally interrupted by an oxygen atom,
Z represents a linear or branched alkyl radical, containing 1 to 8 carbon atoms and optionally substituted, or an aryl or arylalkyl radical, each of these radicals being optionally substituted, in which the alkyl radical contains at most 6 carbon atoms and the aryl radical represents a monocyclic carbocyclic or heterocyclic radical containing 5 or 6 members or a radical constituted by condensed rings containing 8 to 10 members, these radicals optionally containing one or more heteroatoms chosen from oxygen, nitrogen or sulfur atoms,
   the alkyl radicals that can be represented by R'₁₇ and Z, the alkenyl or alkynyl radicals that can be represented by R'₁₇ and the aryl or arylalkyl radicals that can be represented by Z being optionally substituted by one or more radicals chosen from the following radicals:
   halogens,
   amino, alkylamino or dialkylamino in which the alkyl radical or radicals have 1 to 4 carbon atoms,
   hydroxyl,
   free, esterified or salified carboxy,
   alkyl having 1 to 8 carbon atoms, optionally substituted by one or more halogen atoms,
   oxo, cyano, nitro, formyl,
   acyl or acyloxy having at most 12 carbon atoms,
   alkoxy or alkylthio having 1 to 4 carbon atoms,
   carbamoyl,
   alkenyl or alkynyl having at most 4 carbon atoms,
   aryl as defined above,
   and the addition salts of the above.

These compounds possess anti-estrogen activity. They are useful in the treatment of hormonal dependent carcinomas such as for example mammary carcinomas and in the treatment of benign tumors of the breast. S. R. D. Johnston and M. Dowsett, Nature Reviews, Cancer, 3, 821 (nov 2003) describe an alternative strategy in the treatment of hormone-sensitive breast cancer, with aromatase inhibitors, that act through pure estrogen deprivation.

All anti-estrogens are not necessarily pure anti-estrogens as they can have both antagonist and agonist activity. Pure anti-estrogens means estrogen receptor antagonists that have no agonist effect. It has now been evidenced that compounds of formula (I) are pure anti-estrogens.

It has now been found that pure anti-estrogens and preferably 19-nor steroids having a thiocarbonated chain in position 11β can potentiate the effects of aromatase inhibitors.

The combination of a « pure » anti-estrogen and preferably a « pure » anti-estrogen of the formula (I), with aromatase inhibitors leads at least to an additive activity of each compound, as compared to the use of the aromatase inhibitor alone.

According to a preferred embodiment of the invention, among the pure anti-estrogens : 19-nor steroids having a thiocarbonated chain in position 11β, the preferred compounds have the structure of formula (I) wherein :
R₃ is defined as above ;
R₁₆ represents a hydrogen atom ;
R₁₇ and R'₁₇ together form a ketone function or R₁₇ is a hydroxyl radical or an acyloxy radical having at most 6 carbon atoms and R'₁₇ represents a hydrogen atom, an alkyl, alkenyl or alkynyl radical having at most 6 carbon atoms, each of these substituents being optionally substituted by halogens (F, CI) ;
X represents a methylene radical, an arylene or arylenoxy group, having at most 6 carbon atoms linked to the steroid by a carbon atom,
Y represents a saturated or unsaturated, linear aliphatic chain, containing 1 to 6 carbon atoms,
Z represents a linear or branched alkyl radical, containing 1 to 6 carbon atoms and optionally substituted, by one or more halogen atoms, preferably by fluorine.

In the definition of formula (I) according to the European patent EP 623,140 : by acyloxy radical there can be meant in particular the derivative of a saturated or unsaturated aliphatic or cycloaliphatic acid and notably an alkanoic acid such as, for example, acetic, propionic, butyric or isobutyric, valeric or undecylic acid, a hydroxyalkanoic acid such as, for example, hydroxyacetic acid; a cycloalkylcarboxylic or cycloalkylalkanoic acid such as, for example, cyclopropyl, cyclopentyl or cyclohexylcarboxylic, cyclopentyl or cyclohexyl acetic or propionic acid, a benzoic acid, a salicylic acid or a phenylalkanoic acid such as phenyl acetic or phenyl propionic acid, an amino acid such as diethylamino acetic or aspartic acid, formic acid or an optionally salified diacid, such as, for example, butanedioic acid or the monosodium salt of the latter. It is preferably the derivative of acetic, propionic or butyric acid.

By acyl radical there is meant the radicals corresponding to the previous acyloxy radicals.

By alkyl radical there can be meant one of the following radicals: methyl, ethyl, propyl, isopropyl, butyl, isobutyl, n-pentyl, n-hexyl, 2-methyl pentyl, 2,3-dimethyl butyl, n-heptyl, 2-methyl hexyl, 2,2-dimethyl pentyl, 3,3-dimethyl pentyl, 3-ethylpentyl, n-octyl, 2,2-dimethylhexyl, 3,3-dimethylhexyl, 3-methyl 3-ethyl pentyl.

When R₃ represents a cyclic alkyl radical, it can be a cyclopropyl, cyclobutyl, cyclopentyl or cyclohexyl radical. It is preferably the cyclopentyl radical.

By alkoxy radical, there can notably be meant the methoxy, ethoxy, propyloxy, butyloxy radical and by alkylthio radical, there can notably be meant the methylthio, ethylthio, propylthio or butylthio radicals.

By alkenyl radical, there can be meant a vinyl, propenyl, isopropenyl, allyl, 2-methyl allyl, butenyl or isobutenyl radical. It is preferably the vinyl or propenyl radical.

By alkynyl radical, there can be meant the ethynyl, propynyl, propargyl or butynyl radical. It is preferably the ethynyl or propynyl radical.

When R₁₆ represents a halogen atom, it can be a bromine, chlorine, fluorine or iodine atom; it is preferably a bromine atom.

When X represents an arylene group, it is preferably the phenylene radical.

When X represents an arylenoxy group, it is preferably the phenylenoxy radical.

When Y represents a saturated or unsaturated, linear or branched aliphatic chain, it can be one of the following radicals: methylene, ethylene, propylene, isopropylene, butylene, isobutylene, or tert-butylene, n-pentylene, n-hexylene, 2-methyl pentylene, 2,3-dimethyl butylene, n-heptylene, 2-methyl hexylene, 2,2-dimethyl pentylene, 3,3-dimethyl pentylene, 3-ethyl-pentylene, n-octylene, 2,2-dimethyl hexylene, 3,3-dimethyl hexylene, 3-methyl 3-ethyl pentylene, nonylene, 2,4-dimethyl heptylene, n-decylene, n-undecylene, n-dodecylene, n-tridecylene, n-tetradecylene, n-pentadecylene, n-hexadecylene, n-heptadecylene or n-octadecylene, preferably n-pentylene, n-hexylene, n-heptylene, n-octylene or n-nonylene.

It can be a chain interrupted by an oxygen atom, for example, an oxapolymethylene radical, preferably the oxy diethylene radical.

When Z represents a linear or branched alkyl radical, it can be the radicals indicated above, preferably propyl, butyl, n-pentyl.

When Z represents an arylalkyl group, the alkyl radical can be one of the radicals defined above, notably a methyl, ethyl, propyl, isopropyl, butyl, isobutyl, n-pentyl or n-hexyl radical, it is preferably the methyl or ethyl radical.

By aryl group which can be contained in an arylalkyl group, is meant:
a carbocyclic monocyclic radical, for example the phenyl radical,
a heterocyclic monocyclic radical, for example the following radicals: thienyl, furyl, pyrannyl, pyrrolyl, imidazolyl, pyrazolyl, pyridyl, pyrazinyl, pyrimidinyl, pyridazinyl, thiazolyl, oxazolyl, furazannyl, pyrrolinyl, imidazolinyl, pyrazolinyl, thiazolinyl, triazolyl, tetrazolyl, as well as the isomers of position of the heteroatom or heteroatoms that these radicals can contain,
a radical constituted by carbocyclic condensed rings, for example, the naphthyl radical or phenanthrenyl radical,
a radical constituted by heterocyclic condensed rings, for example, benzofurannyl, benzothienyl, benzimidazolyl, benzothiazolyl, naphtho[2,3-b]thienyl, thianthrenyl, isobenzofurannyl, chromenyl, xanthenyl, phenoxathiinyl, indolizinyl, isoindolyl, 3H-indolyl, indolyl, indazolyl, purinyl, quinolizinyl, isoquinolyl, quinolyl, phthalazinyl, naphthyridinyl, quinoxalinyl, quinazolinyl, cinnolinyl, pteridinyl, carbazolyl, beta-carbolinyl, acridinyl, phenazinyl, phenothiazinyl, phenoxazinyl, indolinyl, isoindolinyl, imidazopyridyl, imidazopyrimidinyl or also the condensed polycyclic systems constituted by heterocyclic monocyclics, as defined, for example, above, such as for example furo[2,3-b]pyrrole or thieno[2,3-b]furan.

When Z represents an aryl or arylalkyl group, there can be mentioned as examples of such an aryl radical, in particular, the following radicals: phenyl, furyl such as 2-furyl, imidazolyl such as 2-imidazolyl, pyridyl such as 2-pyridyl, 3-pyridyl or 4-pyridyl, pyrimidinyl such as pyrimid-2-yl, thiazolyl such as thiazol-2-yl, thiazolinyl such as thiazolin-2-yl, triazolyl such as triazol-2-yl, tetrazolyl such as tetrazol-2-yl, benzimidazolyl such as benzimidazol-2-yl, benzothiazolyl such as benzothiazol-2-yl, purinyl such as purin-7-yl or quinolyl such as 4-quinolyl and as examples of arylalkyl radicals, there can also be mentioned in particular the methyl or ethyl radicals substituted by one of the above aryl radicals.

The expression optionally substituted applied to the alkyl radicals that can be represented by R'₁₇ and Z, the alkenyl or alkynyl radicals that can be represented by R'₁₇ and the aryl or arylalkyl radicals that can be represented by Z, indicates that these can be optionally substituted by one or more identical or different radicals chosen from the radicals indicated previously and in particular,
halogen (fluorine, chlorine, bromine, iodine),
amino, alkylamino such as methylamino or ethylamino, dialkylamino such as dimethylamino, diethylamino, methylethylamino,
hydroxyl,
free, esterified carboxy such as alkoxy carbonyl for example methoxy carbonyl or ethoxycarbonyl, or carboxy salified for example by a sodium or potassium atom,
alkyl having 1 to 8 carbon atoms such as methyl, ethyl, propyl, isopropyl, butyl, isobutyl, tert-butyl, optionally substituted by one or more halogen atoms, for example fluorine such as trifluoromethyl,
oxo, cyano, nitro, formyl,
acyl such as acetyl, propionyl, butyryl, benzoyl,
acyloxy such as acetoxy, a radical of formula -O-CO-(CH₂)ₙ-COOH in which n=1 to 5,
alkoxy such as methoxy, ethoxy, propyloxy, isopropyloxy, butyloxy,
alkylthio such as methylthio, ethylthio, propylthio, isopropylthio, butylthio,
carbamoyl,
alkenyl such as vinyl, propenyl,
alkynyl such as ethynyl, propynyl,
aryl such as phenyl, furyl, thienyl.

As an example of such substituted radicals, there can be mentioned for example an alkyl radical substituted by one or more halogen atoms, for example, fluorine, such as the trifluoroethyl, trifluorobutyl, pentafluoropropyl, pentafluorobutyl, pentafluoropentyl, heptafluorobutyl or nonafluorobutyl radical, an alkyl radical substituted by an aryl radical, for example the phenyl radical, itself substituted by one or more halogen atoms and/or alkyl radicals substituted by one or more halogen atoms, such as pentafluorobenzyl, pentafluorophenylethyl, pentafluorophenylpropyl, 4-trifluoromethyl 2,3,5,6-tetrafluorobenzyl.

Among compounds of the formula (I) that can be used in the instant invention a particularly preferred compound is 11β-[4-[5-[(4,4,5,5,5-pentafluoropentyl)sulfonyl]pentyloxy] phenyl]-estra-1,3,5(10)-triene-3,17β-diol.

Among the aromatase inhibitors that can be potentiated with pure anti-estrogens, there can be mentioned : exemestane, atamestane, formestane, fadrozole, triazoles : for example finrozole, anastrozole, letrozole and their derivatives. According to a preferred embodiment of the invention compounds of the formula (I) can be combined with the above mentioned aromatase inhibitors.

Exemestane (6-methylene-androsta-1,4-diene-3,17-dione) has the structure :

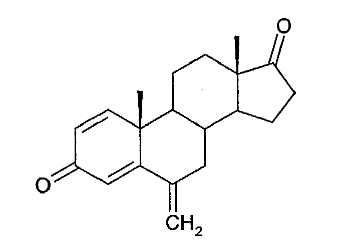

Exemestane is described in more detail in DE 3,622,841 incorporated herein by reference.

Atamestane (1-methyl-androsta-1,4-diene-3,17-dione) has the structure :

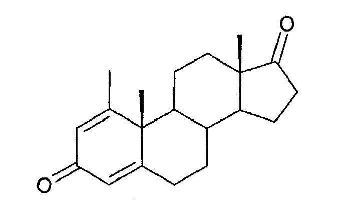

Atamestane is described in more detail in EP 129,500 incorporated herein by reference.

Formestane (4-hydroxy-4-androsten-3,17-dione) and its 4-(C₂-C₁₂)acyloxy derivative :

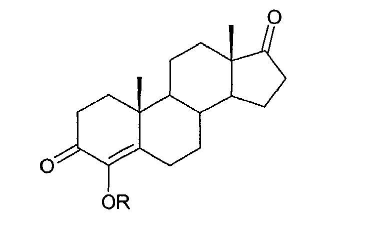

Formestane is described in more detail in US 4,235,893 incorporated herein by reference.

### Fadrozole,

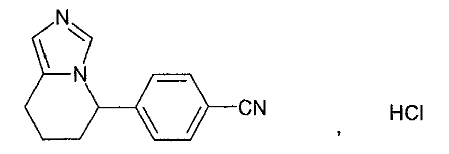

Fadrozole is described in more detail in EP 165,904 incorporated herein by reference.

### Finrozole,

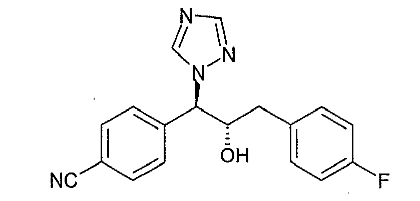

Finrozole is described in more detail in GB 2,273,704 incorporated herein by reference.

### Anastrozole,

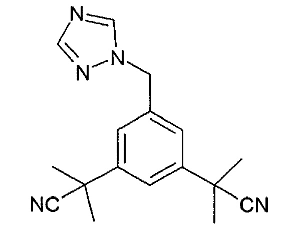

Anastrozole is described in more detail in EP 296,749 incorporated herein by reference.

### Letrozole,

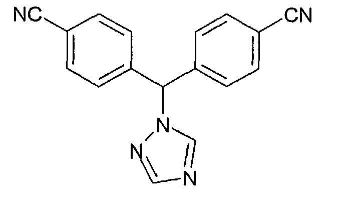

Letrozole is described in more detail in EP 236,940 incorporated herein by reference.

Compounds of formula (I) can be prepared according to EP 623140.

Alternatively, in a prefered embodiment, the amorphous form of 11 β-[4-[5-[(4,4,5,5,5-pentafluoropentyl)sulfonyl]pentyloxy] phenyl]-estra-1,3,5(10)-triene-3,17β-diol prepared according to EP 623140 can be transformed further by crystallisation into Form B through another crystalline form : Form A.

Form B can be defined as follows (wave length : 1.54051 Å) :

| Peak position 2 Theta angle (°) | D space (Å) | relative intensities (%) | Counts number |
|---|---|---|---|
| 3.6890 | 11.9715 | 24.38 | 9978 |
| 3.9890 | 11.0725 | 42.16 | 17259 |
| 4.7590 | 9.2841 | 32.77 | 13414 |
| 5.5290 | 7.9944 | 33.35 | 13652 |
| 6.0190 | 7.3457 | 23.44 | 9596 |
| 6.7690 | 6.5350 | 39.87 | 16322 |
| 7.3290 | 6.0381 | 77.52 | 31731 |
| 7.3890 | 5.9893 | 71.04 | 29080 |
| 7.4890 | 5.9098 | 36.15 | 14798 |
| 7.7790 | 5.6907 | 19.80 | 8105 |
| 8.2590 | 5.3621 | 33.47 | 13701 |
| 8.4490 | 5.2424 | 78.45 | 32114 |
| 8.6190 | 5.1397 | 82.67 | 33841 |
| 9.0490 | 4.8974 | 75.75 | 31006 |
| 9.2590 | 4.7872 | 44.14 | 18069 |
| 9.5690 | 4.6335 | 48.89 | 20013 |
| 10.1490 | 4.3713 | 48.49 | 19850 |
| 10.3890 | 4.2714 | 100.00 | 40934 |
| 10.6890 | 4.1528 | 45.62 | 18673 |
| 10.8390 | 4.0960 | 37.95 | 15534 |
| 11.0490 | 4.0191 | 43.69 | 17883 |
| 11.3190 | 3.9244 | 28.13 | 11513 |
| 11.7590 | 3.7795 | 27.69 | 11335 |
| 12.0590 | 3.6869 | 13.87 | 5677 |
| 12.5590 | 3.5423 | 32.76 | 13412 |
| 13.3390 | 3.3386 | 27.82 | 11388 |
| 13.6190 | 3.2712 | 12.62 | 5166 |
| 13.8990 | 3.2066 | 12.34 | 5050 |
| 14.3490 | 3.1080 | 11.71 | 4793 |
| 15.7690 | 2.8343 | 12.47 | 5105 |
| 16.2090 | 2.7594 | 12.06 | 4935 |
| 16.9190 | 2.6467 | 13.03 | 5335 |
| 17.4090 | 2.5745 | 13.22 | 5412 |
| 18.4290 | 2.4365 | 11.61 | 4753 |
| 18.7690 | 2.3939 | 11.10 | 4542 |
| 21.5590 | 2.0962 | 10.97 | 4492 |

Form A is prepared by crystallisation from amorphous 11β-[4-[5-[(4,4,5,5,5-pentafluoropentyl)sulfonyl]pentyloxy] phenyl]-estra-1,3,5(10)-triene-3,17β-diol in an alcohol such as propanol (i.e. n-propanol, i.propanol) or butanol (i.e. n-butanol, i.butanol, 2-butanol) optionally with the addition of an ether such as isopropyl ether, methyl isobutyl ether, dipropylether, ethyl butyl ether, ethyl isobutyl ether.

The amorphous form of 11β-[4-[5-[(4,4,5,5,5-pentafluoropentyl)sulfonyl]pentyloxy] phenyl]-estra-1,3,5(10)-triene-3,17β-diol is dissolved in a sufficient amount of solvent, the mixture is optionally filtered to get rid of undissolved material, the crystallisation is carried out at room temperature or below (for instance at the temperature of an ice bath) optionally with the addition of an ether, then cooled at a temperature around 0°C. The crystals are collected by filtration, washed and optionally dried under vacum or atmospheric pressure.

The term "sufficient amount" is an amount sufficient to dissolve the amorphous form.

Form B is obtained from Form A, by heating above the melting temperature of Form A. Mainly by heating at the reflux temperature of water or of an organic liquid chosen among those that do not dissolve such Form and which is chosen with a suitable boiling point. The boiling point of the organic liquid must range between the melting points of both polymorphs Form A and Form B, roughly between 100 and 155°C. In these conditions, polymorph A melts in the medium and recrystallizes into the other polymorphic form : Form B. All kinds of organic liquids can be chosen, provided that they have the above mentioned characteristics. Water is a suitable medium for this transformation. The process can be also conducted in a suitable organic liquid or mixtures thereof at a temperature of between 100 and 150°C. The crystallisation process may be carried out under inert atmosphere such as nitrogen atmosphere.

"Suitable organic liquid" means an organic liquid that does not dissolve Form A, has a boiling point between the melting points of both polymorphs A and B and allows the formation of crystals of Form B. The suitable organic liquid can be chosen from organic liquids such as ethers [for instance butyl ether (b.p. = 141°C), methyl heptyl ether (b.p. = 150°C)], or such as linear or branched aliphatic, cycloaliphatic, aromatic or araliphatic hydrocarbons [for instance toluene (b.p. = 111°C), xylene (b.p. # 141°C), cycloheptane (b.p. = 118°C), cyclooctane (b.p. = 150°C), dimethylcyclohexanes (b.p. # 120°C), octane (b.p. = 126°C), isopropylcyclohexane (b.p. = 146°C)].

Form A or Form B is recovered by isolating the crystals, for example by filtration or evaporation of the solvent or for Form B of the organic liquid or water, or by any other method for removing the solvent from the crystals or the crystals from the solvent.

According to a preferred embodiment of the invention are the combinations of pure anti-estrogen with the anti aromatase : letrozole.

According to a most preferred embodiment of the invention are the combinations of the pure anti-estrogen 19-nor steroids having a thiocarbonated chain in position 11β and derivatives of the formula (I) with the anti aromatase : letrozole, and more specifically the combinations of the pure anti-estrogen 11β-[4-[5-[(4,4,5,5,5-pentafluoropentyl)sulfonyl]pentyloxy] phenyl]-estra-1,3,5(10)-triene-3,17β-diol (particularly crystalline Form B) with the anti-aromatase : letrozole.

The activity of the combinations of the invention have been evidenced in the following tests :

### STUDY AIM

DMBA (dimethylbenzanthracene)-induced rat mammary carcinoma is reported to be a very good model for studying the anti-tumor activity of anti-estrogenic agents. The aim of the study was to investigate the *in vivo* antitumor activity of the pure anti-estrogen 11β-[4-[5-[(4,4,5,5,5-pentafluoropentyl)sulfonyl]pentyloxy] phenyl]-estra-1,3,5(10)-triene-3,17β-diol (Form B of said compound is mentioned as "Compound A" in the study described hereafter) after weekly subcutaneous (s.c.) administrations for 4 consecutive weeks (schedule Q7Dx4), alone or in combination with the aromatase inhibitor letrozole after 28 daily *per os* (p.o.) administrations against DMBA-induced ER+ mammary carcinoma in Sprague-Dawley rats.

### MATERIALS & METHODS

Three hundred healthy female Sprague Dawley rats, 7 week-old and weighing 180-220 g were obtained from IFFA CREDO (L'Arbresle, France). Food was provided ad *libitum.*

Compound A (white powder, MW: 658.77 g/mol) is the test substance.

Letrozole (LTZ, Femara®) was supplied as sugar coated tablets containing 2.5 mg of active substance, and lactose, hypromellose, macrogol 8000, corn starch, yellow feroxyde, colloidal silicon dioxide, microcrystalline cellulose, sodium starch carboxymethyl, magnesium stearate as excipients.

The control was the vehicle used for Compound A, an oily mixture for subcutaneous injection, comprising 95% corn oil + 5% ethanol.

### EXPERIMENTAL DESIGN AND TREATMENTS

### Induction of mammary tumors

The DMBA administration at D₀ consisted of a single oral (p.o.) dose of 20.0 mg DMBA/rat. The health status was monitored once a week. The DMBA chemo-induced mammary tumors were checked by palpation of rats once a week.

### Evaluation of antitumor activity after short treatment period

At D₇₄, by which time mammary tumors were palpable, the rats were randomised so that 50% of the rats in each group had tumors, and treatment started. Each group was composed of 15 rats. The treatment regimen was s.c. injections of Compound A at 150 mg/kg/inj, Q7Dx4. p.o. administrations of LTZ at 0.1 mg/kg/adm, Q1 Dx28 alone or in combination with s.c. injections of Compound A at 150 mg/kg/inj, Q7Dx4. Vehicle control was injected s.c. at Q7Dx4.

| **Groups** (15 animals/group) | **Treatment** | **Dose / Route** (mg/kg / adm.) | **Treatment schedule** |
|---|---|---|---|
| 1 | Vehicule control | - / s.c. | Q7Dx4 |
| 2 | Compound A | 150 / s.c. | Q7Dx4 |
| 3 | LTZ | 0.1 / p.o. | Q1 Dx28 |
| 4 | LTZ + Compound A | 0.1 / p.o. and 150/ s.c. | Q1Dx28 and Q7Dx4 |

### Animal monitoring and sacrifice of rats

The location of each tumor among the six mammary complexes of female rats was recorded. The specific mammary gland was identified by site as L- (left) 1 through L-6 and R- (right) 1 through R-6, with 1 being the most cranial and 6 the most caudal.

### RESULTS

### Tumor inhibition systems

The length and width of mammary tumors were measured once a week with callipers and the volume of the tumor estimated by the formula: (length x width²)/2. Treatment efficacy was assessed in terms of the effects of each compound on the tumor volumes of tumor-bearing rats relative to control vehicle-treated rats.

The results were expressed in Figures 1 to 4, as :
**Figure 1 :** Percentage of rats with tumors / group as a function of time,
**Figure 2 :** Total number of palpated tumors / group as a function of time,
**Figure 3 :** Total tumor volume relative to baseline / group as a function of time,
**Figure 4** : Percentage of emerging, growing, stationary, regressing, and disappeared tumors / group as a function of time.
   - Figure 4A shows Tumor Evolution with Control
   - Figure 4B shows Tumor Evolution with Letrozole (LTZ)
   - Figure 4C shows Tumor Evolution with Compound A
   - Figure 4D shows Tumor Evolution with LTZ + Compound A

### CONCLUSION

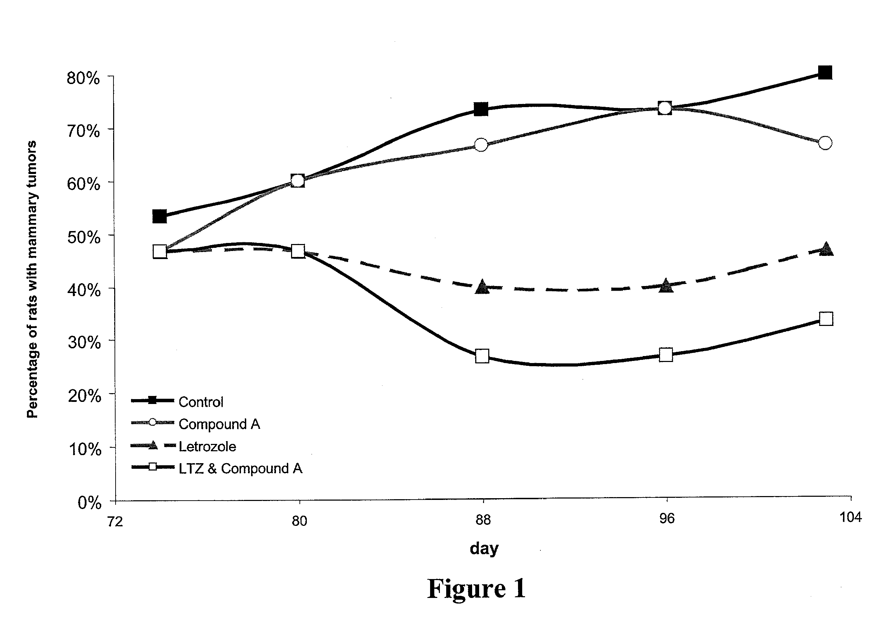

**Figure 1 :** Letrozole alone and the combination of Letrozole with Compound A each decreased the percentage of rats with tumors as compared to Control group. Combination of Letrozole with Compound A presented efficacy better than the additive effects of Letrozole alone and Compound A alone at Day₈₈ and Day₉₆.

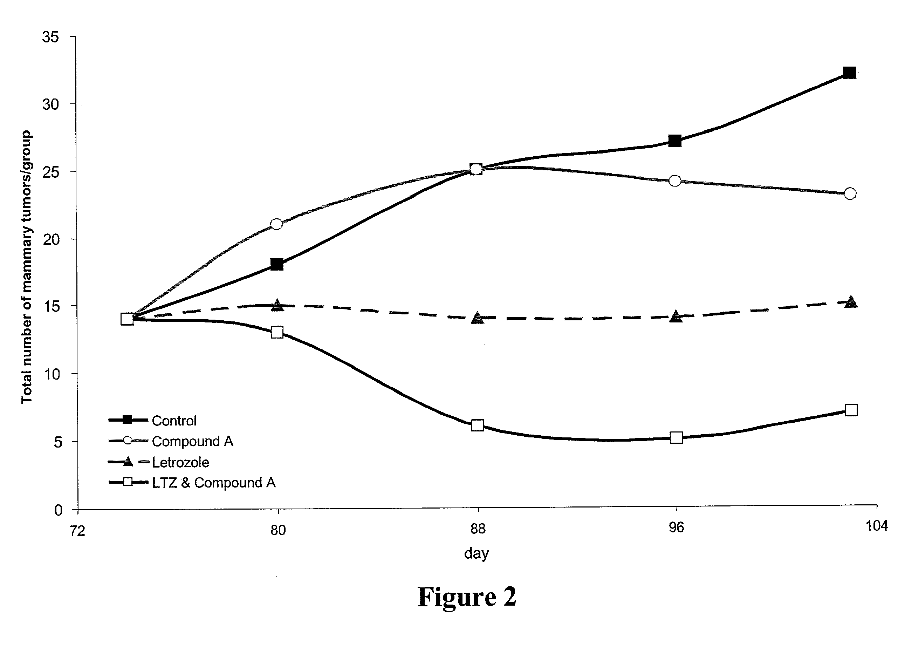

**Figure 2 :** Letrozole alone and the combination of Letrozole with Compound A each decreased the total number of tumors in the group as compared to Control group. Combination of Letrozole with Compound A presented efficacy better than the additive effects of Letrozole alone and Compound A alone at Day₈₈ and Day₉₆ and comparable efficacy at Day₈₀ and Day₁₀₄.

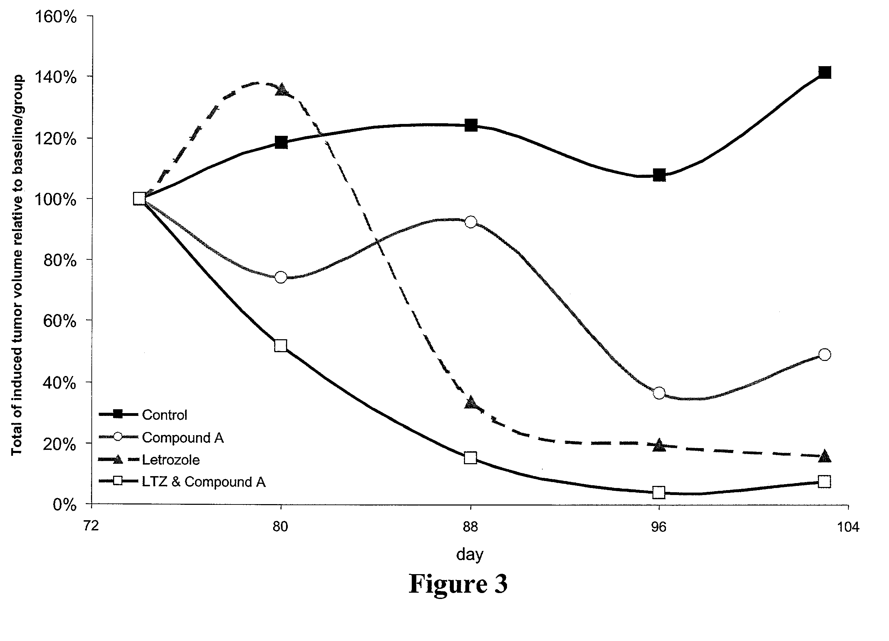

**Figure 3 :** Letrozole alone, Compound A alone and the combination of Letrozole with Compound A each decreased the relative total tumor volume in the group as compared to Control group. Combination of Letrozole with Compound A presented better efficacy as compared to Letrozole alone or Compound A alone. An important decrease in the relative total tumor volume can be observed on Figure 3, with the combination of Letrozole with Compound A, particularly on Day₉₆ and Day₁₀₄ where it is close to 0%.

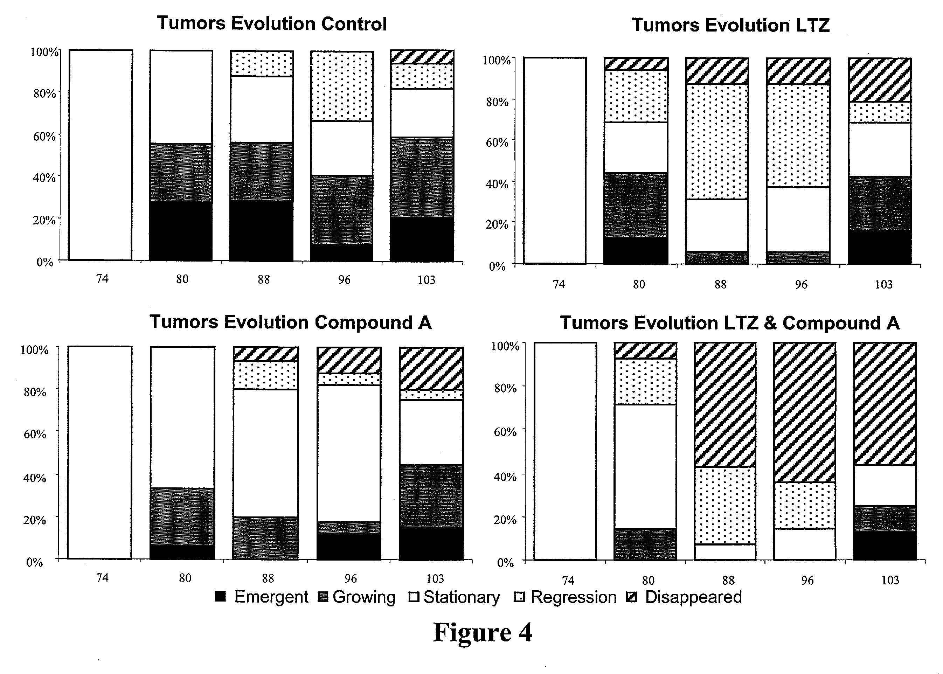

**Figure 4 :** Letrozole alone and the combination of Letrozole with Compound A each had a higher percentage of regressing or disappearing tumors compared to Control group. Combination of Letrozole with Compound A presented efficacy (higher percentage of disappearing tumors) better than the additive effects of Letrozole alone and Compound A alone.

It can be concluded that :

While Letrozole exhibits anti-tumoral effects on the DMBA-induced mammary tumors in Sprague-Dawley rats, the above experiments show that Compound A strongly enhances and potentiates this effect of Letrozole. In the percentage of rats with mammary tumors and in the total number of mammary tumors, and the percentage of disappearing tumors the combination of Letrozole with Compound A presented efficacy better than the additive effects of Letrozole alone and Compound A alone.

### Preparation of compound A :

Amorphous 11β-[4-[5-[4,4,5,5,5-pentafluoropentyl)sulfonyl]pentyloxy]phenyl]-estra-1,3,5(10)-triene-3,17β-diol (86.5 g) is dissolved in isobutanol (200 ml) at 40°C. The hot solution is filtered. On cooling the filtrate an exothermic crystallization occurs. The crystals are collected by filtration, washed with cooled isopropyl ether and dried at 50°C under vacuum in the presence of phosphorous pentoxide. A slightly yellowish white solid of Form A is obtained (72.7 g ; yield 84 %). Melting point: 103 - 104°C.

Form A (61.45 g) is suspended in butyl ether (300 ml) with efficient stirring under an nitrogen atmosphere. It is then heated in an oil bath at 155°C. The compound immediately melts and recrystallizes under the other polymorphic form : Form B. The reflux is maintained with stirring for 30 min in order to turn the agglomerated solid to a white suspension. After cooling the mixture in an ice bath, the compound is collected by filtration, washed with isopropyl ether, then dried at 80 °C under vacuum in the presence of phosphorous pentoxide to give the Form B as white crystals (57.16 g ; yield 93 %). Melting point: 164°C.

In summary, the combination of the pure anti-estrogen : 19-nor steroid having a thiocarbonated chain in position 11β of the formula (I) with an aromatase inhibitor provides a consistent advantage in terms of efficacy. As of today, no other anti-estrogen has been shown to potentiate successfully the activity of aromatase inhibitor.

Generally speaking, the pure anti-estrogen is administered at the usual dose and according to the usual route and scheme of administration used when it is administered alone. Among others, the 19-nor steroid having a thiocarbonated chain in position 11β, or preferably the compound of formula (I) is administered between 25 and 250 mg/kg parenterally by intramuscular or subcutaneous injection, preferably at a dose of between 50 to 250 mg/kg or between 50 and 250 mg/kg by oral route. Preferably the scheme of administration is an injection once a week, weekly, or once a month, monthly. It can also be daily by oral route.
It is also to be understood that other schemes of administration may be adopted.

The aromatase inhibitor is generally administered at the usual dose and according to the usual route and scheme of administration used when it is administered alone. More often it is administered by oral route. The aromatase inhibitor can also be administered by parenteral route such as intramuscular route for example. Generally speaking it is administered at the dose of 1 mg once a day for anastrazole, 25 mg once a day for exemestane, and 2.5 mg once a day for letrozole or by i.m. injection of 250 mg every 2 weeks for formestane. It being understood that the range of administration is adapted to the usual range prescribed for each compound according to the patient. It is also to be understood that other schemes of administration may be adopted.

It is understood that the combination can include several types of administration such as coadministration at the same time or different times.

For the treatment, more often, formulations of the pure anti-estrogen 19-nor steroid having a thiocarbonated chain in position 11β, or preferably the compound of formula (I) are presented in liquid form.
The liquid formulations can be chosen among all suitable formulations known in the art for poorly water soluble compounds, for example solutions in a fatty oil-based vehicule for instance solutions in a ricinoleate vehicule, optionally comprising an alcohol and a non-aqueous ester solvent miscible with the ricinoleate vehicule could be successfully adapted. Solutions in a suitable mixture of water with propylene glycol and PEG such as PEG 400 could also be convenient.
The formulations of the pure anti-estrogen 19-nor steroid having a thiocarbonated chain in position 11β, or preferably the compound of formula (I) can also be presented in solid form such as in the form of tablets, capsules or powder.

More often, the formulations of the aromatase inhibitor are in solid form. These formulations can include tablets or capsules or can include formulations such as powder containing-device. The formulations of the aromatase inhibitor can also be in liquid form suitable for oral or parenteral administration or in the form of powder containing-bottle or vial with a complementary solvent vial for parenteral administration.

As an example, tablets, capsules or powder can include excipients such as monohydrated lactose, colloidal silica, microcristalline cellulose, povidone, sodium carboxymethyl starch, magnesium stearate, Macrogol®, soya lecithin, α-tocopherol and/or sodium hydroxyde.

It will also be understood that the kits of presentation for the formulation of the pure anti-estrogen (preferably the 19-nor steroid having a thiocarbonated chain in position 11β) and the formulation of the aromatase inhibitor also fall within the present invention. Presentation kits of any form can be suitable, for example presentation in the form of a syringe or an infusion bag comprising the pure anti-estrogen (preferably the 19-nor steroid having a thiocarbonated chain in position 11β) and a tablet, a capsule or a bottle and/or a vial comprising the aromatase inhibitor, or presentations involving bottles or vials of each of the compounds or bottles comprising one compound and vials comprising the second compound or alternatively presentations involving bottles, vials, tablets or capsules comprising the pure anti-estrogen (preferably the 19-nor steroid having a thiocarbonated chain in position 11β) and a tablet, a capsule or a bottle and/or a vial comprising the aromatase inhibitor.

More preferably, it will also be understood that the kits of presentation that include the formulation of the pure anti-estrogen of the formula (I) and the formulation of the aromatase inhibitor also fall within the present invention. Presentation kits of any form can be suitable, for example presentation in the form of a syringe or an infusion bag comprising the anti-estrogen of the formula (I) and a tablet, a capsule or a bottle and/or a vial comprising the aromatase inhibitor, or presentations involving bottles or vials of each of the compounds or bottles comprising one compound and vials comprising the second compound or alternatively presentations involving bottles, vials, tablets or capsules comprising the anti-estrogen of the formula (I) and a tablet, a capsule or a bottle and/or a vial comprising the aromatase inhibitor.

## Claims

1. A combination of pure anti-estrogen, with aromatase inhibitors.

2. A combination according to claim 1 of a pure anti-estrogen 19-nor steroid having a thiocarbonated chain in position 11β, with aromatase inhibitors.

3. A combination according to claim 1 or 2, wherein the 19-nor steroid having a thiocarbonated chain in position 11β has the formula : 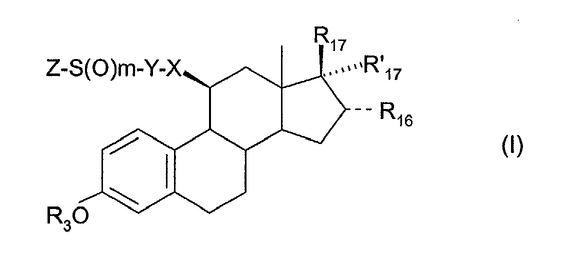 in which
R₁₇ and R'₁₇ are such that:
either R₁₇ and R'₁₇ together form a ketone function, an oxime function, a hydrazono function or a methylene radical,
or R₁₇ is a hydroxyl radical, a hydroxymethyl radical or an acyloxy radical having at most 12 carbon atoms and R'₁₇ represents a hydrogen atom, an alkyl, alkenyl or alkynyl radical having at most 8 carbon atoms, each of these substituents being optionally substituted,
R₃ represents a hydrogen atom, a linear or branched alkyl radical or a cyclic alkyl radical having at most 8 carbon atoms or an acyl radical having at most 12 carbon atoms,
R₁₆ represents a hydrogen atom, a halogen atom or an alkyl radical having at most 8 carbon atoms,
m has the value 0, 1 or 2,
X, Y and Z are such that:
X represents a methylene radical, an arylene or arylenoxy group, having at most 10 carbon atoms linked to the steroid by a carbon atom,
Y represents a saturated or unsaturated, linear or branched aliphatic chain, containing 1 to 18 carbon atoms, optionally interrupted by an oxygen atom,
Z represents a linear or branched alkyl radical, containing 1 to 8 carbon atoms and optionally substituted, or an aryl or arylalkyl radical, each of these radicals being optionally substituted, in which the alkyl radical contains at most 6 carbon atoms and the aryl radical represents a monocyclic carbocyclic or heterocyclic radical containing 5 or 6 members or a radical constituted by condensed rings containing 8 to 10 members, these radicals optionally containing one or more heteroatoms chosen from oxygen, nitrogen or sulfur atoms,
the alkyl radicals that can be represented by R'₁₇ and Z, the alkenyl or alkynyl radicals that can be represented by R'₁₇ and the aryl or arylalkyl radicals that can be represented by Z being optionally substituted by one or more radicals chosen from the following radicals:
halogens,
amino, alkylamino or dialkylamino in which the alkyl radical or radicals have 1 to 4 carbon atoms,
hydroxyl,
free, esterified or salified carboxy,
alkyl having 1 to 8 carbon atoms, optionally substituted by one or more halogen
atoms,
oxo, cyano, nitro, formyl,
acyl or acyloxy having at most 12 carbon atoms,
alkoxy or alkylthio having 1 to 4 carbon atoms,
carbamoyl,
alkenyl or alkynyl having at most 4 carbon atoms,
aryl as defined above,
and the addition salts of the above.

4. A combination according to claim 3, wherein in formula (I)
R₃ is defined as in claim 3 ;
R₁₆ represents a hydrogen atom ;
R₁₇ and R'₁₇ together form a ketone function or R₁₇ is a hydroxyl radical or an acyloxy radical having at most 6 carbon atoms and R'₁₇ represents a hydrogen atom, an alkyl, alkenyl or alkynyl radical having at most 6 carbon atoms, each of these substituents being optionally substituted by halogens ;
X represents a methylene radical, an arylene or arylenoxy group, having at most 6 carbon atoms linked to the steroid by a carbon atom,
Y represents a saturated or unsaturated, linear aliphatic chain, containing 1 to 6 carbon atoms,
Z represents a linear or branched alkyl radical, containing 1 to 6 carbon atoms and optionally substituted, by one or more halogen atoms.

5. A combination according to any of claims 1 to 4, wherein the pure anti-estrogen is the 11β-[4-[5-[(4,4,5,5,5-pentafluoropentyl)sulfonyl]pentyloxy] phenyl]-estra-1,3,5(10)-triene-3,17β-diol.

6. A combination according to claim 5, wherein the pure anti-estrogen is the crystalline Form B of 11β-[4-[5-[(4,4,5,5,5-pentafluoropentyl)sulfonyl]pentyl-oxy] phenyl]-estra-1,3,5(10)-triene-3,17β-diol

7. A combination according to any of claims 1 to 6, wherein the aromatase inhibitor is chosen from exemestane, atamestane, formestane, fadrozole, triazoles and derivatives thereof.

8. A combination according to claim 7, wherein triazoles are chosen from finrozole, anastrozole, letrozole and derivatives thereof.

9. A combination according to claims 1, 7 or 8, wherein a pure anti-estrogen is combined with the aromatase inhibitor letrozole.

10. A combination according to any of claims 1 to 9, wherein the pure anti-estrogen 11 β-[4-[5-[(4,4,5,5,5-pentafluoropentyl)sulfonyl]pentyloxy] phenyl]-estra-1,3,5(10)-triene-3,17β-diol is combined with the aromatase inhibitor letrozole.

11. A combination according to claim 10, wherein the pure anti-estrogen 11β-[4-[5-[(4,4,5,5,5-pentafluoropentyl)sulfonyl]pentyloxy] phenyl]-estra-1,3,5(10)-triene-3,17β-diol, crystalline Form B, is combined with the aromatase inhibitor letrozole.

12. A combination according to claim 1, wherein the pure anti-estrogen and the aromatase inhibitor are coadministered at the same time or administered at different times.

13. A combination according to any of claim 1 to 11, wherein the pure anti-estrogen 19-nor steroid having a thiocarbonated chain in position 11β and the aromatase inhibitor are coadministered at the same time or administered at different times.

14. A combination according to any of claim 1 to 11, wherein the pure anti-estrogen 19-nor steroid having a thiocarbonated chain in position 11β is administered by parenteral or oral route.

15. A combination according to any of claim 1 to 11, wherein the aromatase inhibitor is administered orally or parenterally.

16. Kit of presentation of a combination according to any of claims 1 to 11, wherein the presentation is in the form of a syringe or an infusion bag comprising the pure anti-estrogen and a tablet, a capsule or a bottle and/or a vial comprising the aromatase inhibitor, or presentations involving bottles or vials of each of the compounds or bottles comprising one compound and vials comprising the second compound or alternatively the presentation is in a form involving involving bottles, vials, tablets or capsules comprising the pure anti-estrogen and a tablet, a capsule or a bottle and/or a vial comprising the aromatase inhibitor.

17. Kit of presentation according to claim 16, wherein the presentation is in the form of a syringe or an infusion bag comprising the pure anti-estrogen 19-nor steroid having a thiocarbonated chain in position 11β and a tablet, a capsule or a bottle and/or a vial comprising the aromatase inhibitor, or presentations involving bottles or vials of each of the compounds or bottles comprising one compound and vials comprising the second compound or alternatively the presentation is in a form involving involving bottles, vials, tablets or capsules comprising the pure anti-estrogen 19-nor steroid having a thiocarbonated chain in position 11β and a tablet, a capsule or a bottle and/or a vial comprising the aromatase inhibitor.

18. Kit of presentation according to claim 17, wherein the presentation is in the form of a syringe or an infusion bag comprising the pure anti-estrogen of the formula (I) according to claim 3, and a tablet, a capsule or a bottle and/or a vial comprising the aromatase inhibitor, or presentations involving bottles or vials of each of the compounds or bottles comprising one compound and vials comprising the second compound or alternatively the presentation is in a form involving bottles, vials, tablets or capsules comprising the pure anti-estrogen of the formula (I) according to claim 3, and a tablet, a capsule or a bottle and/or a vial comprising the aromatase inhibitor.

19. Kit of presentation according to any of claims 16 to 18, wherein the pure anti-estrogen is 11β-[4-[5-[(4,4,5,5,5-pentafluoropentyl)sulfonyl]pentyloxy] phenyl]-estra-1,3,5(10)-triene-3,17β-diol or its crystalline Form B.

20. Kit of presentation according to claim 16, wherein the aromatase inhibitor is letrozole.

21. Kit of presentation according to any of claim 19 or 20, wherein the pure anti-estrogen is 11β-[4-[5-[(4,4,5,5,5-pentafluoropentyl)sulfonyl]pentyloxy] phenyl]-estra-1,3,5(10)-triene-3,17β-diol or its crystalline Form B and the aromatase inhibitor is letrozole.
